# EUROPÄISCHE PATENTSCHRIFT

(11) **EP 2 455 377 B1**
(45) Veröffentlichungstag und Bekanntmachung des Hinweises auf die Patenterteilung: **09.07.2014**
(21) Anmeldenummer: 10190813.5
(22) Anmeldetag: 11.11.2010
(51) Int. Cl.: C07D 409/06, C07D 401/06, A61K 31/4535, A61K 31/454

(54) **Synthese von Fentanyl Analoga**
Synthesis of fentanyl analogs
Synthèse d'analogues de fentanyl

(43) Veröffentlichungstag der Anmeldung: 23.05.2012
(73) Patentinhaber: hameln rds gmbh, 31789 Hameln (DE)
(72) Erfinder: Kakalík, Ivan, 900 81 Senkvice (SK); Králová, Janka, 900 01 Modra (SK); Valachovic, Pavol, 902 01 Pezinok (SK); Smahovský, Vendel, 902 01 Pezinok (SK)

(56) Entgegenhaltungen:
- US-A- 4 179 569
- US-A1- 2010 016 365

## Beschreibung

### Gebiet der Erfindung

Die vorliegende Erfindung betrifft ein Verfahren zur Herstellung von Opioid-Analgetika und Anästhetika und deren Vorstufen. Insbesondere betrifft die vorliegende Erfindung einen alternativen Prozess für die Synthese von Alfentanil und Sufentanil.

### Stand der Technik

Die Piperidinderivate Sufentanil und Alfentanil Citrat gehören zu einer Familie von hochwirksamen synthetischen Opioiden.

Sufentanil, (N-[4-(Methoxymethyl)-1[2-(2-thienyl) ethyl-4-piperidinyl]-N-phenylpropanamide 2-hydroxy-1,2,3-propanetricarboxylate) der Summenformel C₂₈H₃₈N₂O₉S mit einem Molekulargewicht von 578.68g/mol wurde erstmals 1974 synthetisiert (Niemegeers et al. Arzneim. Forsch. 1976, 26, 1551-1556).

Sufentanil wird als starkes Analgetikum mit ausgezeichneter Sicherheitsmarge im Vergleich zu anderen Betäubungsmitteln beschrieben. Des Weiteren bindet es mit hoher Affinität und Spezifität an den µ-Opioid Rezeptor. Im Gegensatz zu Fentanyl und Morphin führt Sufentanil zu einer vollständigen Anästhesie mit minimalen Nebenwirkungen. Aufgrund seiner geringen kardiovaskulären Toxizität, wird Sufentanil Citrat bevorzugt für größere chirurgische Eingriffe, die eine vollständige intravenöse Anästhesie erfordern, eingesetzt. Alfentanil, N-1[1-[2-(4-Ethyl-4,5-dihydro-5-oxo-1H-tetrazol-1-yl) ethyl]-4-(methoxymethyl)-4-piperidinyl]-N-phenylpropanamid, ist ein weiteres Analgetikum aus der Reihe der Fentanyl-Analoga. Laut einer Veröffentlichung in Drugs of Today (1984, 20 (1), 7-13) wird Alfentanil als nahezu ideales Analgetikum mit Eigenschaften wie zuverlässiger Wirksamkeit, dosisabhängiger Analgesie und raschem Wirkeintritt angesehen. Des Weiteren ist die Wirkdauer flexibel an die klinischen Anforderungen anpassbar, die kardiovaskuläre Beeinträchtigung wird als minimal angesehen und der Patient kann sich schnell und vollständig von der Analgesie erholen. Die analgetische Potenz von Alfentanil beträgt ein Viertel der Potenz von Fentanyl und die Wirkdauer von Alfentanil liegt bei etwa einem Drittel der einer äquianalgetischen Dosis an Fentanyl. Hingegen zeichnet sich Alfentanil durch einen zur äquianalgetischen Dosis viermal schnelleren Wirkeintritt aus. Schneller Wirkeintritt und kurze Wirkdauer machen Alfentanil besonders geeignet für kürzere operative Eingriffe (Sinclair und Cooper, Anaesthesia, 1983, 38, 435-437).

Die Synthese von Sufentanil ist in der Patentschrift US 3998834 (1975) von Janssen offenbart. Das dort beschriebene Verfahren ist jedoch sehr langwierig und kompliziert. Die Synthese verläuft über insgesamt 10 Schritte mit einer geringen Gesamtausbeute von etwa 2% (Schema 1).

Die gleiche Synthese wurde von G. Henriksen et al und P.G.H. van Daele ausführlich beschrieben. (G. Henriksen et al, J.Med.Chem, 48, 7720 bis 7732 (2005) und (P.G.H. Van Daele et al, Arzneim Forsch. 26, (8), 1521-1531 (1976)).

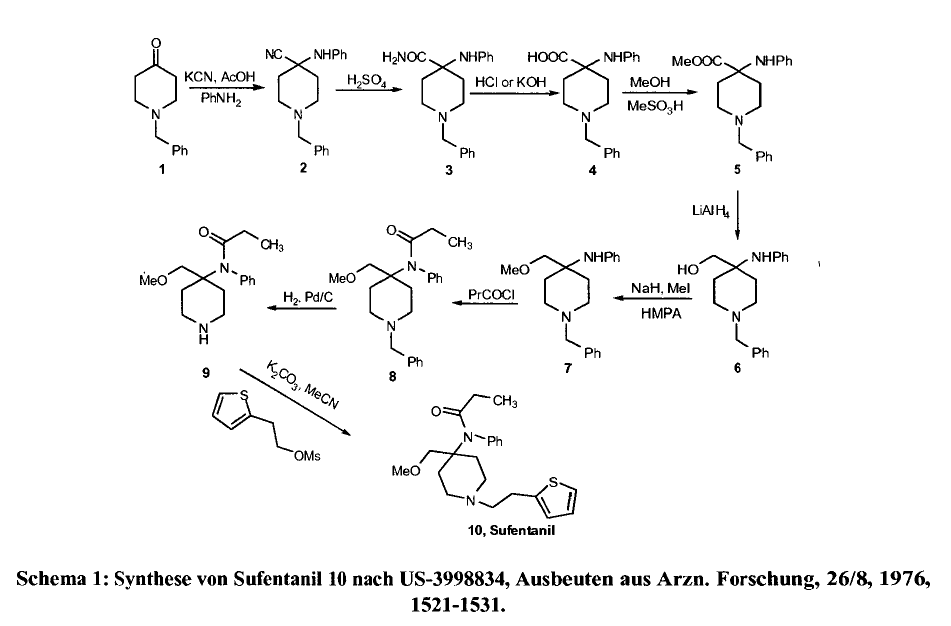

Eine Verbesserung der Synthese durch Optimierung der Umwandlung von Nitril 2 zu Ester 5 wurde in US-Patent 5489689 von Mallinckrodt veröffentlicht.

Die Janssen Synthese von Sufentanil und Alfentanil beginnt mit einer Strecker Kondensation von N-Benzyl-4-piperidon 1 und Anilin in Gegenwart von Kaliumcyanid. Das resultierende Cyanoamin 2 wird anschließend in konzentrierter Schwefelsäure zu Amid 3 hydrolysiert und anschließend durch Erhitzen in Salzsäure zu Carbonsäure 4 umgesetzt. Veresterung gefolgt von einer Reduktion des entstandenen Esters 5 mit Lithiumaluminiumhydrid ergibt 4-(Hydroxymethyl)-4-anilino-N-benzylpiperidin 6. Methylierung des Alkohols 6 und anschließende Acylierung mit Propionylchlorid ergibt Amid 8. Nach Hydrogenolyse der N-Benzyl-Schutzgruppe entsteht das sekundäre Amin 9, welches mit der Thiophen-Seitenkette zu Sufentanil 10 alkyliert wird. Durch Einführung einer Tetrazol-Seitenkette im letzten Schritt der Synthese kann über die gleiche Sequenz auch Alfentanil 35 dargestellt werden.

Die von Janssen entwickelte Synthese von Sufentanil und Alfentanil wird durch die Tendenz des intermediären Piperidins 9, beim Stehen in Lösung einer intramolekularen Acyl-Wanderung zu unterliegen, begrenzt (Schema 2). Dies, ebenso wie eine Acyl-Wanderung vom Sauerstoff zum Stickstoff wurde bereits von Colapret beschrieben (J. A. Colapret et al, J. Med. Chem., 32, 968 (1989)).

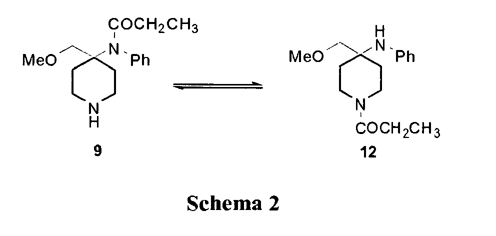

WO 2008/005423 beschreibt eine Optimierung der Ursprungssynthese aus US 3998834. Die hier beschriebene Synthesesequenz unterscheidet sich nur im dritten Schritt, wo anstelle der freien Säure direkt das Natriumsalz 13 isoliert und anschließend alkyliert wird (Schema 3). Die Gesamtausbeute erhöht sich auf 15%.

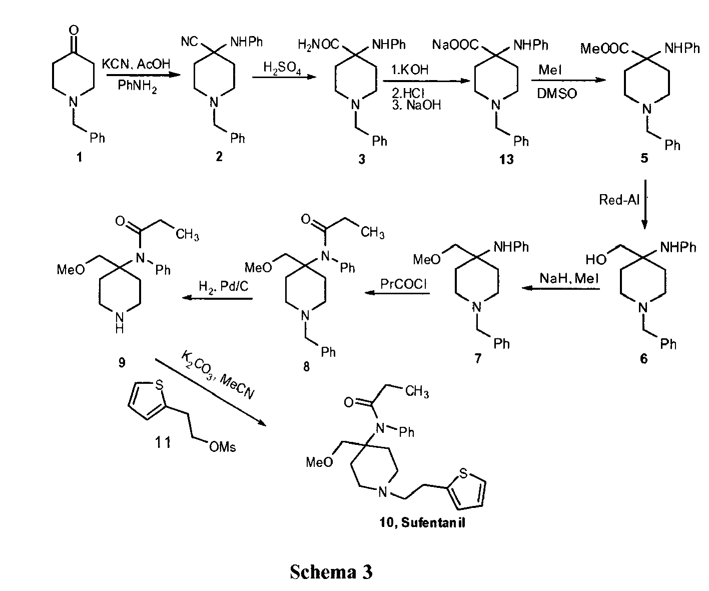

Eine weitere Möglichkeit für die Synthese von Sufentanil ist in Patentschrift WO 9509152 von Mallinckrodt Chemical beschrieben (Schema 4). Die Reaktionssequenz beginnt mit der Umsetzung von Piperidon 14 mit Chloroform. Als Zwischenprodukt wird Epoxid 15 gebildet, welches mit Anilin weiter zu 4-Amino-4-carboxyamino-piperidin 16 reagiert. Im nächsten Schritt wird unter basischen Bedingungen die Schutzgruppe am Stickstoff entfernt und das entstandene 4-(Phenylamino)-4-piperidincarboxanilin 17 wird alkyliert.

Das gleiche Verfahren wird ebenfalls in US 5489689 (1996) von Mallinckrodt Chemical beschrieben. Beide Patente (WO 9509152 (1993) und US 5489689 (1996)) beanspruchen das Verfahren entsprechend Schema 4 mit besonderem Fokus auf der Synthese von Amid 16 und dessen Umwandlung zu Hydroxyl-Derivat 20. Die Gesamtausbeute dieser Synthese liegt ebenfalls bei etwa 15 %, die Reinheit wird mit 99 % angegeben.

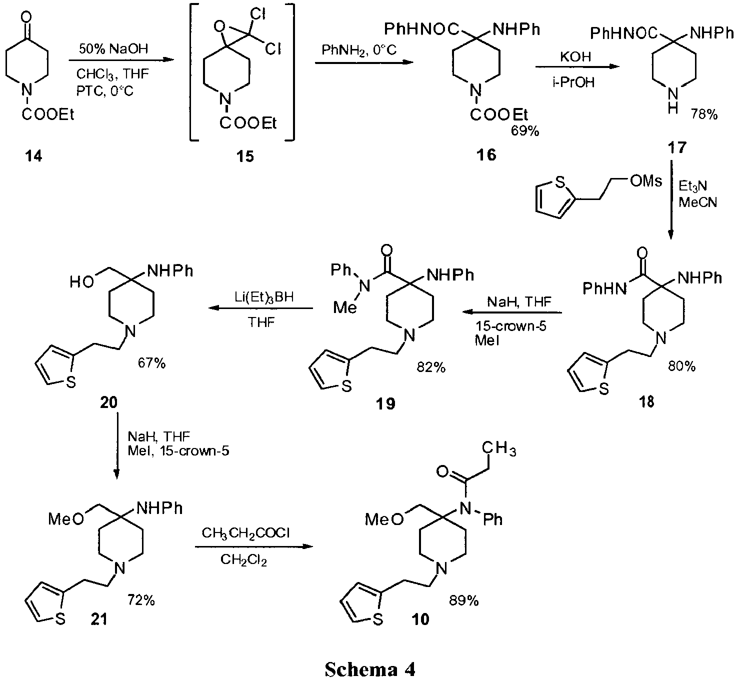

Eine Variante der in WO 9509152 beschriebenen Synthese wird in den Patenten EP 1246801 (1999), US 20040138461 (2004) und US 20060149071 (2006) von Mallinckrodt Chemical offenbart (Schema 5). Die ersten beiden Schritte der Synthese sind identisch. Im dritten Schritt wird Intermediat 16 unter basischen Bedingungen mit Methyliodid geschützt. Die Ethylformiat-Schutzgruppe von Derivat 22 wird anschließend durch Hydrolyse mit Natronlauge entfernt und die Carbonsäureamidgruppe von 23 wird zu Alkohol 24 reduziert. Die Darstellung des Intermediats 20 erfolgt durch Alkylierung des Piperidinstickstoffs von 24 mit 2-(2-Thienyl)-ethanolmethansulfonat. Die letzten Schritte werden analog der bereits in Schema 4 beschriebenen Synthese aus WO9509152 durchgeführt. Die Gesamtausbeute liegt bei etwa 16% und das daraus resultierende Sufentanil hat eine Reinheit von 99%.

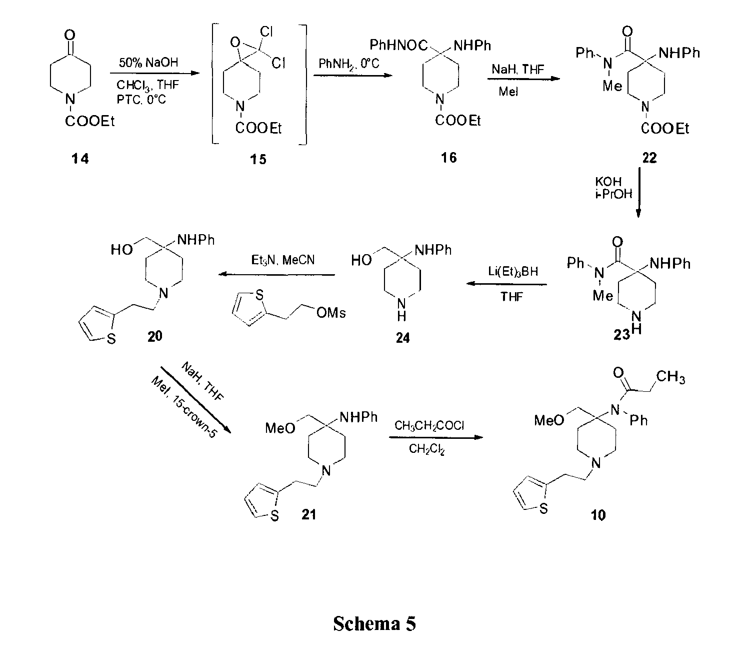

In (Arch. Pharm. Res.: 22 (4), 398-400 (1999)) wird die Synthese von Sufentanil über eine sechsstufige Sequenz beschrieben (Schema 6). Die Schlüsselschritte dieser Synthese bestehen zum einen im effizienten Aufbau des Thiophenylethylpiperidons 29 und zum anderen in der regioselektiven, nucleophilen Ringöffnung von Epoxid 30 mit Anilin unter Lewis-Säure Katalyse. Die Methylierung des gebildeten Piperidylmethanols 20 wird durch Umsetzung mit Diazomethan erreicht. Schließlich wird die Synthese durch Acylierung von Anilinopiperidin 21 mit Propionsäureanhydrid abgeschlossen. Diese Synthese kann nur im Labormaßstab eingesetzt werden.

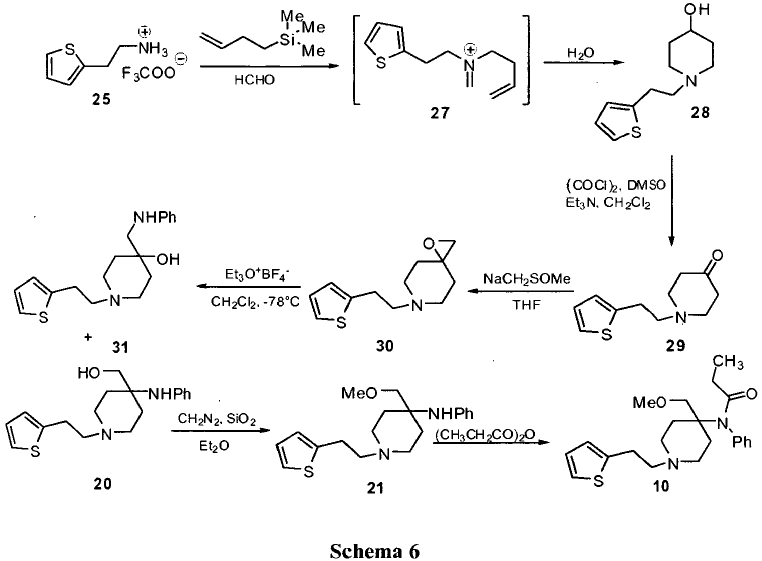

### Beschreibung der Erfindung

Verschiedene 1-substituierte 4-[(1-Oxoalkyl)phenylamino)-4-(methoxymethyl)]piperidin-Derivate sowie verschiedene Möglichkeiten der Synthese sind im Stand der Technik beschrieben.

Wir haben nun gefunden, dass es speziell für die Synthese von AlfentaniLund Sufentanil vorteilhaft ist den Acylsubstituenten und den Substituenten am Piperidin-Stickstoff in einem späten Schritt der Synthese einzuführen. Das Intermediat 32 spielt dabei eine zentrale Rolle.

Die vorliegende Erfindung beschreibt die Synthese und Verwendung des Intermediats I (Schema 7) für die Herstellung von Alfentanil und Sufentanil.
Bei Verwendung von Verbindung 32 als Zwischenprodukt zur Synthese von Alfentanil und Sufentanil werden Ausbeuten von bis zu 35% bzw. 43% über fünf Stufen erzielt.

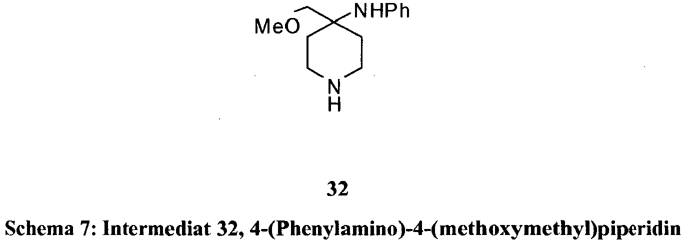

Das neue Intermediat 32 ermöglicht die Synthese von Alfentanil und Sufentanil in einer übersichtlichen Zwei-Stufensequenz (Schema 8), was zu hohen Produktausbeuten führt.

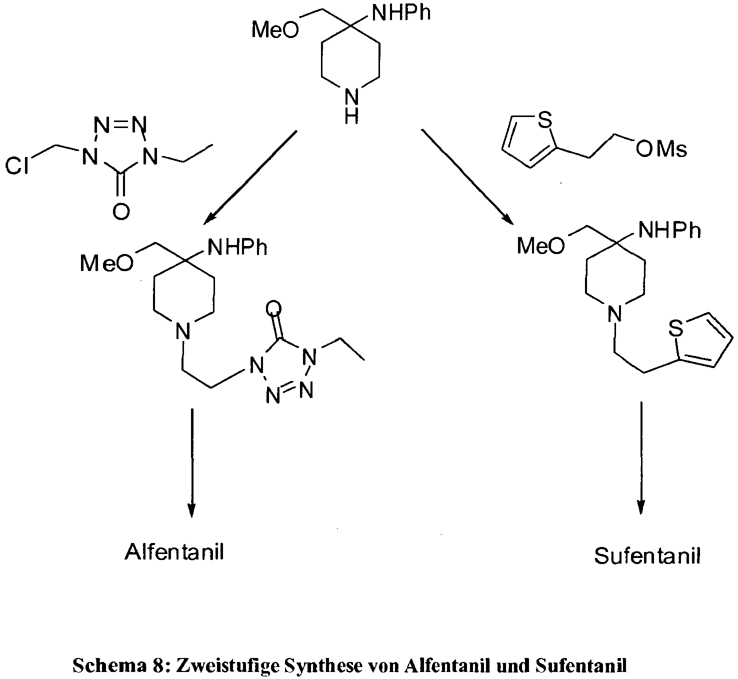

Der Vorteil der Synthese nach Schema 8 ist die Vermeidung einer Acyl-Wanderung nach Schema 2, da die Acylgruppe erst im letzten Schritt der Synthese eingefügt wird, und somit nie ein freies Pyrimidin-Stickstoff parallel zu einem Acylierten Anilin-Stickstoff im Molekül existiert. Trotzdem ist die Synthese synergetisch günstig, da erst in einer sehr späten Phase der Synthese die verschiedenen Substituenten am Pyridyl-Stickstoffatom installiert werden.

In einer bevorzugten Ausgestaltung der Erfindung wird.Sufentanil daher in folgenden Schritten synthetisiert:
a) Umsetzen von 4-(Phenylamino)-4-(methoxymethyl)piperidin mit 2-(2-Thienyl)-ethanol-methansulfonat, so dass 4-Methoxymethyl-4(phenylamino)-1-(2-thienyl)-ethyl-piperidin gebildet wird: 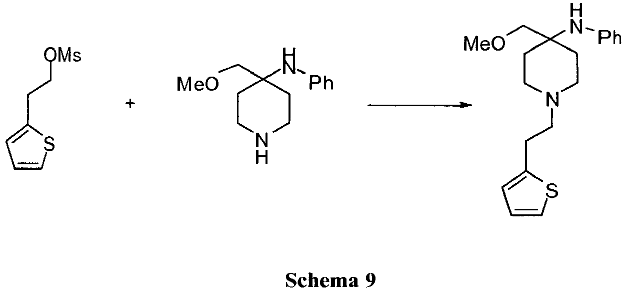
b) Umsetzen von 4-Methoxymethyl-4(phenylamino)-1-(2-thienyl)-ethyl-piperidin mit einem Acylierungsreagenz, so dass Sufentanil gebildet wird:

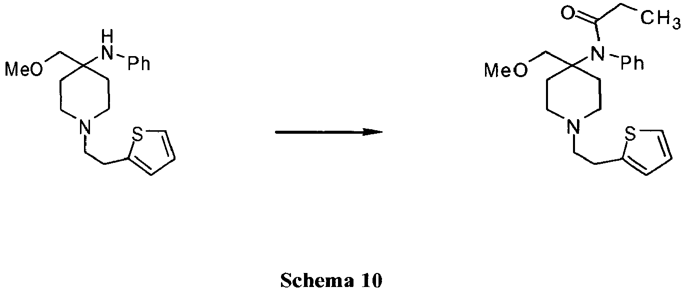

Für das Alkylierungsmittel 11 sind auch andere Abgangsgruppen als Methansulfonat möglich, was aber nicht zur Verbesserung der Ausbeute führt.

Die besten Ausbeuten für die Umsetzung ergeben sich für die Umsetzung von 4-(Phenylamino)-4-(methoxymethyl)-piperidin' m i t 2-(2-Thienyl)-ethanol-methansulfonat, wenn sie in Gegenwart eines Lösungsmittels ausgewählt aus Acetonitril, 2-Propanol, 2-Butanol, Butan-2-on, und Isobutylmethylketon oder deren Mischungen durchgeführt wird.

Es hat sich gegenüber anderen Lösungsmitteln gezeigt, dass es zu erhöhten Ausbeuten, kommt, wenn die Umsetzung von 4-(Phenylamino)-4-(methoxymethyl)-piperidin mit 2-(2-Thienyl)-ethanol-methansulfonat in in Gegenwart eines der oben genannten Lösungsmittel in einem Temperaturbereich zwischen 50 °C und 108 °C durchgeführt wird.

In einer bevorzugten Ausgestaltung der Erfindung wird die Reaktion von 4-(Phenylamino)-4-(methoxymethyl)piperidin mit 2-(2-Thienyl)-ethanol-methansulfonat in in Gegenwart eines der oben genannten Lösungsmittel in einem Temperaturbereich zwischen 60 °C und 80 °C durchgeführt.

In einer besonders bevorzugten Ausgestaltung der Erfindung wird die Reaktion von 4-(Phenylamino)-4-(methoxymethyl)-piperidin mit 2-(2-Thienyl)-ethanol-methansulfonat in Gegenwart eines der oben genannten Lösungsmittel bei der Siedetemperatur des Reaktionsgemisches unter Atmosphärendruck durchgeführt.

Es hat sich weiterhin gezeigt, dass hohe Ausbeuten mit großem Reinheitsgrad erzielt werden können, wenn das 4-Methoxymethyl-4-(phenylamino)-1-(2-thienyl)-ethyl-piperidin, das nach einer der oben genannten Methoden hergestellt wurde durch Kristallisation gereinigt wird. Hilfsweise kann eine chromatographische Reinigung angewendet werden.

In einer bevorzugten Ausgestaltung der Erfindung wird das 4-Methoxymethyl-4(phenylamino)-1-(2-thienyl)-ethyl-piperidin das nach einer der oben genannten Methoden hergestellt wurde durch Kristallisation in einem Alkohol gereinigt. Es hat sich im Gegensatz zu anderen verwendeten Lösungsmitteln gezeigt, dass die Kristallisation in einem Alkohol höhere Ausbeuten eine erhöhte Produktqualität ergibt.

In einer weiteren Ausgestaltung der Erfindung wird die Reaktion von 4-Methoxymethyl-4(phenylamino)-1-(2-thienyl)-ethyl-piperidin mit Propionsäurechlorid oder Propionsäureanhydrid in einem polaren Lösungsmittel durchgeführt.

In einer bevorzugten Ausgestaltung der Erfindung wird die Reaktion von 4-Methoxymethyl-4(phenylamino)-1-(2-thienyl)-ethyl-piperidin mit Propionsäurechlorid oder Propionsäureanhydrid in einem Lösungsmittel, ausgewählt aus der Gruppe von Dimethylformamid, Dichlormethan, Dimethylsulfoxid und Tetrahydrofuran durchgeführt. In anderen Lösungsmitteln hat sich teilweise gezeigt, dass die Reaktionszeiten verlängert, oder die Ausbeuten gering sind.

In einer besonders bevorzugten Ausgestaltung der Erfindung wird die Reaktion von 4-Methoxymethyl-4(phenylamino)-1-(2-thienyl)-ethyl-piperidin mit Propionsäurechlorid oder Propionsäureanhydrid in Dichlormethan durchgeführt.

Eine weitere Verwendung von 4-(Phenylamino)-4-(methoxymethyl)piperidin ist die Umsetzung zu Alfentanil. Alfentanil wird nach der Lehre der vorliegenden Erfindung durch die Umsetzung von 1-Ethyl-4-(2-(4-methoxymethyl)-4-(phenylamino)piperidin-1-yl)ethyl)-1H-tetrazol-5(4H)-on mit Propionsäurechlorid oder Propionsäureanhydrid hergestellt.

In einer bevorzugten Ausgestaltung der Erfindung wird das 1-Ethyl-4-(2-(4-methoxymethyl)-4-(phenylamino)piperidin-1-yl)ethyl)-1H-tetrazol-5(4H)-on hergestellt durch die Reaktion von 4-(Phenylamino)-4-(methoxymethyl)-piperidin mit einem Alkylierungsmittel ausgewählt aus s 1-(2-Chlorethyl)-4-ethyl-1,4-dihydro-5H-tetrazol-5-on, 1-(2-Bromethyl)-4-ethyl-1,4-dihydro-5H-tetrazol-5-on, 1-(2-lodoethyl)-4-ethyl-1,4-dihydro-5H-tetrazol-5-on oder 1-(2-Methansulfonat-ethanoyl)-4-ethyl-1,4-dihydro-5H-tetrazol-5-on.

In einer besonders bevorzugten Ausgestaltung der Erfindung wird das 1-Ethyl-4-(2-(4-methoxymethyl)-4-(phenylamino)piperidin-1-yl)ethyl)-1H-tetrazol-5-(4H)-on hergestellt durch die Reaktion von 4-(Phenylamino)-4-(methoxymethyl)-piperidin mit 1-(2-Methansulfonat-ethanoyl)-4-ethyl-1,4-dihydro-5H-tetrazol-5-on. Andere Alkylierungsmittel zeigten geringere Ausbeuten.

In einer weiteren bevorzugten Ausgestaltung der Erfindung wird das 1-Ethyl-4-(2-(4-methoxymethyl)-4-(phenylamino)piperidin-1-yl)ethyl)-1H-tetrazol-5(4H)-on hergestellt durch Umsetzen von 4-(Phenylamino)-4-(methoxymethyl)piperidin mit 1-(2-Methansulfonat-ethanoyl)-4-ethyl-1,4-dihydro-5H-tetrazol-5-on in einem Lösungsmittel ausgewählt aus der Gruppe von Acetonitril, 2-Propanol, 2-Butanol, Butan-2-on, Methylisobutylketon und deren Mischungen. Auch hier zeigten sich gegenüber der Verwendung anderer Lösungsmittel vergleichsweise bessere Ausbeuten und höhere Reinheitsgerade.

In einer besonders bevorzugten Ausgestaltung der Erfindung wird das 1-Ethyl-4-(2-(4-methoxymethyl)-4-(phenylamino)piperidin-1-yl)ethyl)-1H-tetrazol-5(4H)-on hergestellt durch Umsetzen von 4-(Phenylamino)-4-(methoxymethyl)piperidin mit 1-(2-Methansulfonat-ethanoyl)-4-ethyl-1,4-dihydro-5H-tetrazol-5-on in einem Lösungsmittel ausgewählt aus der Gruppe von Acetonitril, 2-Propanol, 2-Butanol, Butan-2-on, Methylisobutylketon und deren Mischungen bei der Siedetemperatur des Reaktionsgemisches.

Erfindungsgemäß hergestellte Fentanyl-Derivate sind sehr rein. In einer Ausgestaltung der Erfindung beträgt daher die Summe der Verunreinigungen von Sufentanil oder Alfentanil, hergestellt nach den Verfahren der vorliegenden Erfindung, weniger als 0,05%.

Das Intermediat 32 wird nach einer bevorzugten Ausführungsform der Erfindung nach folgendem Schema hergestellt:

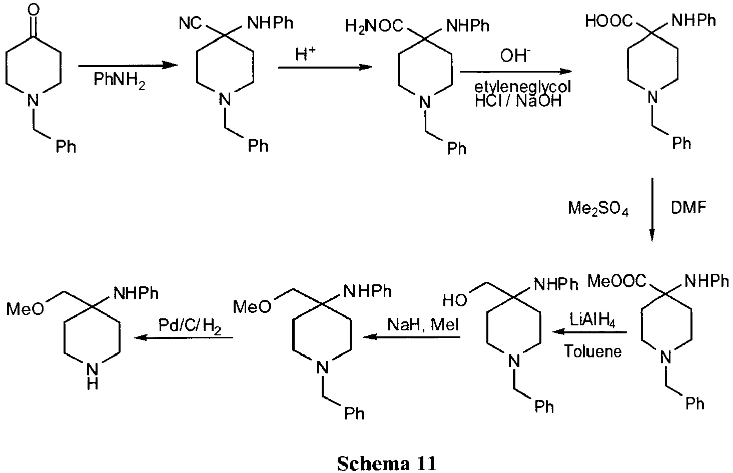

Die Ausbeute dieser Synthese beträgt 48 %.

In einer bevorzugten Ausführungsform wird 4-(Phenylamino)-4-(methoxymethyl) piperidin 32 in einer siebenstufigen Synthese ausgehend von N-Benzylpiperidon 1 dargestellt.

In einer besonders bevorzugten Ausführungsform der Erfindung wird hierzu zunächst N-Benzyl-Piperidon 1 in einer Strecker Kondensation zu Cyanoamin 2 umgesetzt, welches im Folgenden zu Amid 3 hydrolisiert wird. Unter basischen Bedingungen wird N-Benzyl-4carboxy-4-anilinopiperidin Natriumsalz 13 isoliert. N-Benzyl-4-carboxy-4-anilinopiperidin Natiumsalz 13 wird dann mit Dimethylsulfat in DMF alkyliert und mit Lithiumaluminiumhydrid in Toluol zu 4-Hydroxy-4-anilino-N-benzylpiperidin 6 reduziert.

Für die Methylierung wurden neben der bevorzugten Ausführungsform folgende Bedingungen verwendet:

| Bedingungen | Ausbeute |
|---|---|
| MeI/DMF/50°C/1 hour then R.T. overnight | Zersetzung |
| MeI/IDMF/50°C/0.5 hour | Nebenprodukte |
| MeI/DMSO/50°C/0.5 hour | Kaum Produkt |
| MeI/DMF/50°C/15 min. | 68% |
| MeI/DMF/50°C/15 min. | 40% nach Aufreinigung |
| Me2SO4/DMF/50°C/15 min. | 71% |
| Me2SO4/DMF/ from 70°C to R.T./15 min | 77% |
| Me2CO3/DMF/ from 70°C to R.T./15 min | Keine Reaktion |
| Me2SO4/DMF/ from 70°C to R.T./15 min | 77% |
| Me2SO4/DMF/ R.T./0.5hour | 76% |

Alkylierung mit Natriumhydrid und Methyliodid liefert 4-(Hydroxymethyl)-4-anilino-N-benzylpiperidin 7, aus welchem anschließend durch Hydrogenolyse der Schutzgruppe am Piperidin-Stickstoff zu 4-(Hydroxymethyl)-4-anilino-N-benzylpiperidin erhalten wird.

Für die Methylierung des Alkohols wurden folgende Bedingungen getestet:

| Bedingungen | Ausbeute |
|---|---|
| 10 N NaOH/toluene/R.T./20 hours/Me₂SO₄/PTC-catalyst | Keine vollständige Umsetzung |
| NaH/DMF/6 hours / MeI/R.T./ overnight | Keine vollständige Umsetzung |
| NaH/THF/6 hours/ MeI/R.T./ overnight | 72% |
| NaH/DMF/6 hours/50°C /MeI/ overnight | 0% |
| NaH/THF/6 hours/50°C / MeI/ overnight | 30% |
| K2CO3/ACN/MeI/reflux | 0% |
| NaH/THF/0°C/6 hours/ MeI/0°C/overnight | 75% |
| NaH/DMSO/R.T./5 hours / MeI/R.T./overnight | 79% |
| KOH/DMSO/MeI/R.T./overnight | 0% |
| KOtBu/THF/MeI/R.T./overnight | 34% |
| NaH/THF/MeI/R.T./1 hour / 1. equiv. 15-crown-5 | 84% |
| NaH/THF/R.T./1 hour / 0.1 equiv. 15-brown-5 or 18-crown-6 | Keine vollständige Umsetzung |
| NaH/THF/R.T./5 hours / MeI/1.5 hour/R.T. | 81% |
| NaH/THF//R.T./5 hours / Me₂SO₄/1.5 hours | 85% |
| 1.3 equiv.NaH/THF/R.T./5hours / Me₂SO₄/R.T.-30°C/3hours | 83% |
| 1.2 equiv.NaH/THF/5°C/4.5hours / Me₂SO₄/THF/10-30°C/2.5hours | 95% |

In einer anderen bevorzugten Ausführungsform der Erfindung wird Verbindung 32 nach fol-gendem Schema hergestellt:

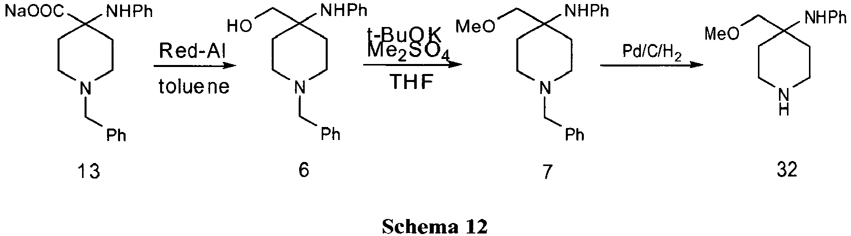

In einer besonders bevorzugten Ausführungsform wird 4-(Phenylamino)-4-(methoxymethyl) piperidin 32 in einer dreistufigen Synthese aus N-Benzyl-4-carboxy-4-anilinopiperidin Natiumsalz dargestellt.

In einer besonders bevorzugten Ausführungsform der Erfindung wird N-Benzyl-4-carboxy-4-anilinopiperidin Natiumsalz 13 zunächst mit Red-Al in Toluol zu 4-(Hydroxymethyl)-4-anilino-N-benzylpiperidin 6 umgesetzt, welches im nächsten Schritt mit Dimethylsulfat und Kalium-tert.-butylat unter Erhalt von 4-(Hydroxymethyl)-4-anilino-N-benzylpiperidin 7 methyliert wird. Verbindung 32 wird anschließend durch Hydrogenolyse der N-Benzylschutzgruppe mit Pd/C und Wasserstoff synthetisiert.

In einer besonders bevorzugten Ausführungsform der Erfindung werden Alfentanil und Sufentanil nach folgendem Schema hergestellt:

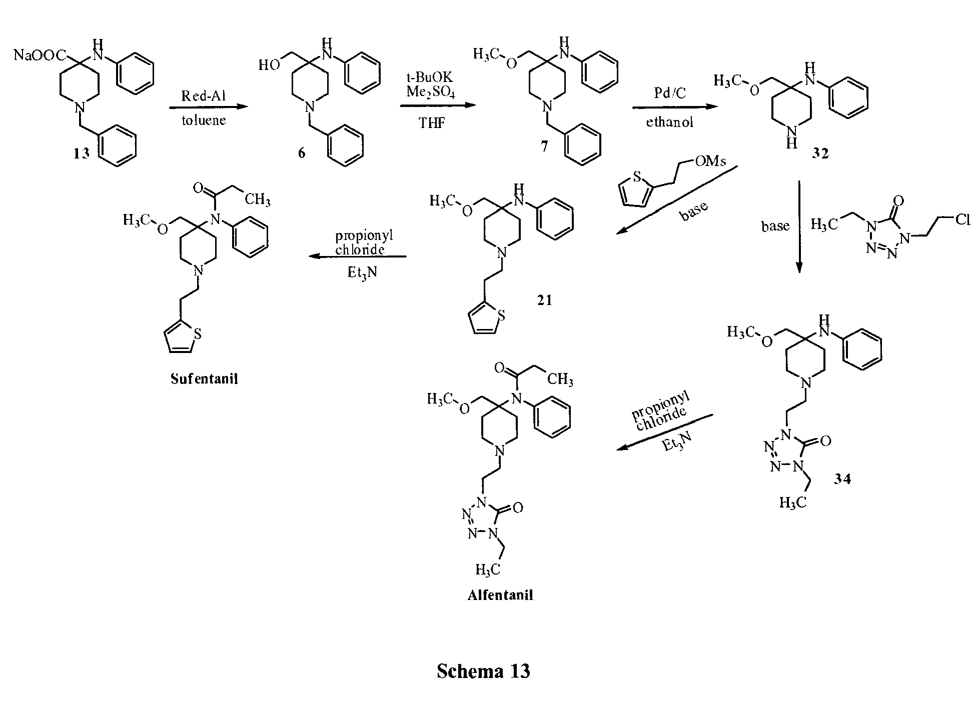

Die Erfindungsgemäß hergestellten Wirkstoffe werden bevorzugt in pharmazeutischen Zubereitungen verabreicht. Die Wirkstoffe können in allen im Stand der Technik bekannten Zubereitungen und für die im Stand der Technik beschriebenen Indikationen verwendet werden.

### Beispiele

### Beispiel 1

### Synthese von 1-Benzyl-4-phenylamino-4-(hydroxymethyl)piperidin

Natrium-1-benzyl-4-(phenylamino)piperidin-4-carbonsäure (93,1 g, 0,282 mol) wird in 1240 ml Toluol durch kräftiges Rühren suspendiert. Die Mischung wird anschließend auf 45 °C erhitzt und Red Al (250 g, 0,843 mol) wird unter Stickstoffatmosphäre zugegeben. Das Reaktionsgemisch wird für 1 Stunde auf 90 °C erwärmt. Die Mischung wird dann auf Raumtemperatur abgekühlt und eine 20% ige Lösung von Natrium-Kalium-Tartrat (930 ml) wird hinzugefügt. Die Mischung wird kräftig gerührt und die organische Phase wird abgetrennt. Die wässrige Phase wird anschließend zwei Mal mit 150 ml Toluol extrahiert und die organischen Phasen werden vereinigt. Die vereinigten organischen Phasen werden mit Kochsalzlösung und mit Wasser gewaschen und anschließend über Natriumsulfat getrocknet. Der Feststoff wird abfiltriert und die Lösung zur Trockene eingedampft. Das Rohprodukt wird dann in 450 ml Methanol aufgenommen, und mit gasförmiger Salzsäure (15%) gesättigt. 200 ml Methanol werden abdestilliert. Die Lösung wird nun auf Eis gekühlt und gerührt, bis das Produkt auskristallisiert. Das Produkt wird abfiltriert und 5 Stunden unter Vakuum getrocknet [1-Benzyl-4-(phenylamino) piperidin-4-yl] methanol Dihydrochlorid 95,4 g (92%), 98,9% Reinheit nach HPLC.
¹H NMR (300MHz, CDCl₃) δ (ppm): 1.66 (2H, m), 1.89 (2H, m), 2.27 (2H, m), 2.59 (3H, m), 3.31 (1H, bs), 3.49 (2H, s), 3.60 (2H, s), 6.76 (2H, d), 6.82 (1H, t), 7.16 (2H, m), 7.27 (5H, m)
MS: m/e =297 (M+1)⁺

### Beispiel 2

### Synthese von 1-Benzyl-4-phenylamino-4-(hydroxymethyl)piperidin

Natrium-1-benzyl-4-(phenylamino)piperidin-4-carbonsäure (10 g, 0,0303 mol) wird in 130 ml Toluol suspendiert. Die Mischung wird bei Raumtemperatur unter kräftigem Rühren unter Stickstoff-Atmosphäre mit Lithiumaluminiumhydrid (1,4 g, 0,04 mol) versetzt. Das Reaktionsgemisch wird für 3 Stunden auf 70°C erhitzt, bevor die Mischung auf Raumtemperatur abgekühlt und eine 20% ige Lösung von Natrium-Kalium-Tartrat (100 ml) hinzugefügt wird. Die Mischung wird anschließend kräftig gerührt und die organische Phase wird abgetrennt. Die wässrige Phase wird zwei Mal mit 20 ml Toluol extrahiert und die organischen Phasen werden vereinigt. Die vereinigten organischen Phasen werden nun mit Kochsalzlösung und Wasser gewaschen und über Natriumsulfat getrocknet. Der Feststoff wird abfiltriert und die Lösung zur Trockene eingedampft. Das Rohprodukt wird dann in 50 ml Propan-2-ol aufgenommen, und mit gasförmiger Salzsäure (15%) gesättigt. Die Lösung wird auf Eis abgekühlt und gerührt, bis das Produkt auskristallisiert. Das Produkt wird abfiltriert und 7 Stunden unter Vakuum getrocknet.
[1-Benzyl-4-(phenylamino) piperidin-4-yl] methanol Dihydrochlorid 9,7 g (88%), 98,5% Reinheit nach HPLC.
¹H NMR (300MHz, CDCl₃) δ (ppm): 1.66 (2H, m), 1.89 (2H, m), 2.27 (2H, m), 2.59 (3H, m), 3.31 (1H, bs), 3.49 (2H, s), 3.60 (2H, s), 6.76 (2H, d), 6.82 (1H, t), 7.16 (2H, m), 7.27 (5H, m)
MS: m/e =297 (M+1)⁺

### Beispiel 3

### Synthese von 1-Benzyl-4-phenylamino-4-(methoxymethyl)piperidin

Natriumhydrid (37,5 g, 0,936 mol) wird durch kräftiges Rühren unter Stickstoffatmosphäre in 600 ml Tetrahydrofuran suspendiert. Die Mischung wird auf 8 °C abgekühlt und 1-Benzyl-4-phenylamino-4-(hydroxymethyl)-piperidin (231 g, 0,780 mol) in 650 ml THF werden tropfenweise zu der Reaktionsmischung über einen Zeitraum von 40 Minuten zugegeben. Nachdem die Zugabe beendet ist, wird 4,5 Stunden bei Raumtemperatur gerührt. Dann wird Dimethylsulfat (81,4 ml, 0,860 mol) in 80 ml THF der Suspension über einen Zeitraum von 40 Minuten zugegeben und bei Raumtemperatur für wird für weitere 2 Stunden gerührt. Destilliertes Wasser (600 ml) wird nun langsam zur Reaktionsmischung gegeben und anschließend wird die Suspension dreimal mit je 300 ml Toluol extrahiert. Die organischen Phasen werden vereinigt und mit 200 ml Kochsalzlösung und 200 ml Wasser gewaschen. Natriumsulfat wird hinzugefügt, um Feuchtigkeit aus der Lösung zu entfernen. Der Feststoff wird abfiltriert und die Lösung wird zur Trockene eingedampft. Das Produkt wird dann unter Vakuum für 5 Stunden getrocknet, um 214 g (83%) eines hellgelben-braunen Öls mit 97,3% Reinheit nach HPLC zu erhalten.
¹H NMR (300MHz, CDCl₃) δ (ppm): 1.74 (2H, m), 1.92 (2H, m), 2.47 (2H, dt), 2.56 (2H, m), 3.30 (3H, s), 3.32 (2H, s), 3.52 (2H, s), 6.81 (3H, m), 7.15 (2H, m), 7.28 (5H, m)
MS: m/e =311 (M+1)⁺

### Beispiel 4

### Synthese von 1-Benzyl-4-phenylamino-4-(methoxymethyl)piperidin

Kalium-tert-butylat (87,5 g, 0,936 mol) wird in 700 ml Tetrahydrofuran durch kräftiges Rühren unter Stickstoffatmosphäre suspendiert. Die Mischung wird auf 8 °C gekühlt und 1-Benzyl-4-phenylamino-4-(hydroxymethyl)piperidin (231 g, 0,780 mol) in 700 ml THF wird tropfenweise zu der Reaktionsmischung über einen Zeitraum von 10 Minuten gegeben. Nachdem die Zugabe beendet ist, wird noch 5 Stunden bei 30 °C gerührt. Dimethylsulfat (81,4 ml, 0,860 mol) in 80 ml THF wird zu der Suspension über einen Zeitraum von 30 Minuten unter Rühren gegeben und bei Raumtemperatur wird für weitere 2 Stunden gerührt. Destilliertes Wasser (700 ml) wird anschließend langsam zur Reaktionsmischung hinzugefügt und die Suspension wird dreimal mit je 300 ml Toluol extrahiert. Die organischen Phasen werden mit 300 ml Kochsalzlösung und 300 ml Wasser gewaschen. Natriumsulfat wird zugegeben, um Feuchtigkeit aus der Lösung zu entfernen. Der Feststoff wird abfiltriert und die Lösung zur Trockene eingedampft. Das Produkt wird unter Vakuum für 5 Stunden getrocknet 237,1 g (92%) eines hellgelb-braunen Öls werden erhalten. Die Reinheit des Produktes 98,5% nach HPLC.
¹H NMR (300MHz, CDCl₃) δ (ppm): 1.74 (2H, m), 1.92 (2H, m), 2.47 (2H, dt), 2.56 (2H, m), 3.30 (3H, s), 3.32 (2H, s), 3.52 (2H, s), 6.81 (3H, m), 7.15 (2H, m), 7.28 (5H, m)
MS: m/e =311 (M+1)⁺

### Beispiel 5

### Synthese von 4-phenylamino-4-(methoxymethyl)piperidin

Eine Hydrierapparatur wird mit 1-Benzyl-4-phenylamino-4-(methoxymethyl)piperidin (50 g, 0,161 mol), 10% Palladium auf Kohle (2,5 g) und 225 ml Methanol beschickt. Die Reaktionsmischung wird bei 8 bar und 45 °C über einen Zeitraum von 3,5 Stunden hydriert. Nach der Reaktion wird das Gemisch auf Raumtemperatur abgekühlt und filtriert. Der Katalysator wird mit 50 ml Methanol gewaschen und die Lösung wird zur Trockne eingedampft. Das flüssige Produkt wird unter Vakuum getrocknet um 35,4 g (99,8%) Rohprodukt zu erhalten. Die HPLC-Analyse ergab 98,9% Reinheit.
¹H NMR (300MHz, CDCl₃) δ (ppm): 1.64 (2H, m), 1.87 (2H, m), 2.83 (2H, dt), 3.03 (2H, dt), 3.30 (3H, s), 3.32 (2H, s), 6.82 (3H, m), 7.15 (2H, m)
¹³C NMR, δ: 33.77 (2xCH₂), 41.98 (2xCH₂), 55.57 (C), 59.04 (CH₃O), 76.87 (CH₂O), 119.81 (CHₐᵣ), 119.93 (2xCHₐᵣ), 128.78 (2xCHₐᵣ), 145.83 (Cₐᵣ)
MS: m/e =221 (M+1)⁺

### Beispiel 6

### Synthese von 4-Phenylamino-4-(methoxymethyl)piperidin

Eine Hydrierapparatur wird mit 1-Benzyl-4-phenylamino-4-(methoxymethyl)piperidin (10 g, 0,032 mol), 10% Palladium auf Kohle (0,5 g) und 45 ml Methanol beschickt. Die Reaktionsmischung wird bei 1 bar, bei Raumtemperatur über einen Zeitraum von 10 Stunden hydriert. Nach der Reaktion wird das Gemisch filtriert. Der Katalysator wird mit 10 ml Methanol gewaschen und die Lösung wird zur Trockne eingedampft. Das flüssige Produkt wird unter Vakuum getrocknet um 6.9 g (97.2 %) Rohprodukt zu erhalten. Die HPLC-Analyse ergab 98.4% Reinheit.
¹H NMR (300MHz, CDCl₃) δ (ppm): 1.64 (2H, m), 1.87 (2H, m), 2.83 (2H, dt), 3.03 (2H, dt), 3.30 (3H, s), 3.32 (2H, s), 6.82 (3H, m), 7.15 (2H, m)
¹³C NMR, δ: 33.77 (2xCH₂), 41.98 (2xCH₂), 55.57 (C), 59.04 (CH₃O), 76.87 (CH₂O), 119.81 (CHₐᵣ), 119.93 (2xCHₐᵣ), 128.78 (2xCHₐᵣ), 145.83 (Cₐᵣ)
MS: m/e =221 (M+1)⁺

### Beispiel 7

### Synthese von 4-(Methoxymethyl)-N-phenyl-1-[2-(thiophen-2-yl)ethyl]piperidin-4-amin

Eine Mischung aus 100,5 g (0,456 mol) 4-Phenylamino-4-(methoxymethyl)piperidin, 103,5 g (0,502 mol) 2-(Thiophen-2-yl)ethylmethansulfonat, 53,2 g (0,502 mol) Natriumcarbonat in 1300 ml Methylisobutylketon wird 28 Stunden unter Rückfluss gerührt. Die Reaktionsmischung wird auf 25 °C abgekühlt und zur Trockne eingeengt. Das Rohprodukt wird in einer Mischung aus 400 ml destilliertem Wasser und 750 ml Ethylacetat gelöst und die organische Phase wird abgetrennt und mit 30 ml Wasser gewaschen. Eine Lösung von 80 ml Methansulfonsäure in 800 ml destilliertem Wasser wird in die Lösung von Ethylacetat gegossen und bei bei 30 °C über 20 Minuten kräftig gerührt. Das Wasser wird abgetrennt, mit 100 ml Ethylacetat gewaschen und tropfenweise in 550 ml 2N Natronlauge gegeben. Der weiße Niederschlag wird mit 3 x 250 ml Ethylacetat extrahiert und die vereinigten organischen Phasen werden mit Natriumsulfat getrocknet und zur Trockne eingedampft. Das Rohprodukt wird aus 900 ml 70% Propan-2-ol kristallisiert um 106 g zu erhalten (70,3%) 4-(Methoxymethyl)-N-phenyl-1-[2-(thiophen-2-yl)ethyl]piperidin-4-amin mit einer Reinheit von 99,86% nach HPLC. ¹H NMR (300MHz, CDCl₃) δ (ppm): 1.78 (2H, m), 1.99 (2H, m), 2.55 (2H, m), 2.70 (4H, m), 3.03 (2H, t), 3.31 (3H, s), 3.33 (2H, s), 3.48 (1H, br), 6.82 (3H, m), 6.90 (1H, d), 7.11 (1H, dd), 7.13 (1H, d), 7.18 (2H, m)
¹³C NMR, δ: 27.83 (CH₂), 32.79 (2xCH₂), 49.04 (2xCH₂), 55.09 (C), 59.13 (CH₃O), 60.09 (CH₂N), 76.90 (CH₂O), 119.79 (3xCHₐᵣ), 123.48 (CHₐᵣ), 124.59 (CHₐᵣ), 126.59 (CHₐᵣ), 128.86 (2xCHₐᵣ), 147.81 (Cₐᵣ), 145.91 (Cₐᵣ)
MS: m/e =331 (M+1)⁺

Andere mögliche Reaktionsbedingungen werden in folgender Tabelle zusammengefasst:

| **Bedingungen** | **Base** | **Time** **(hours)** | **Conversion** **HPLC (%)** |
|---|---|---|---|
| Acetonitrile / 80-85 °C | Et₃N | 10 | 78.2 |
| 2-Propanol / 80-85 °C | Na₂CO₃ | 20 | 81.4 |
| 2-Butanol / 100-110 °C | Na₂CO₃ | 6 | 96.3 |
| Butan-2-on (MEK) / 75-85 °C | Na₂CO₃ | 10 | 61.7 |
| Butan-2-on (MEK) / 75-85 °C | Et₃N | 10 | 68.8 |
| Butan-2-on (MEK) / 75-85 °C | - | 10 | 51.6 |
| Butan-2-on (MEK) / 75-85 °C | NaHCO₃ | 6 | 62.0 |
| Butan-2-on (MEK) / 75-85 °C | K₂CO₃ | 21.5 | 86.1 |
| Butan-2-on (MEK) / 75-85 °C | Na₂CO₃ | 22.5 | 87.4 |

### Beispiel 8

### Herstellung von N-(4-(Methoxymethyl)-1-(2-(2-thienyl)-ethyl)-4-piperidinyl)-N-phenyl-propanamid

4-(Methoxymethyl)-N-phenyl-1-(2-(thiophen-2-yl)ethyl)piperidin-4-amin 45 g (0,136 mol), 400 ml Dichlormethan (CH₂Cl₂) und 38 ml (0,272 mol) Triethylamin werden in einem 2 L, 3-Hals-Glaskolben vorgelegt. 15,4 ml (0,177 mol) Propionylchlorid wird tropfenweise zu der Reaktionsmischung über einen Zeitraum von 40 Minuten hinzugefügt. Nach der Zugabe wird die Reaktionsmischung bei Raumtemperatur für 3 Stunden gerührt. Dann wird das Reaktionsgemisch mit 400 ml destilliertem Wasser verdünnt und die organische Phase wird von der wässrigen Phase getrennt. Die organische Phase wird mit 10% iger Natriumcarbonatlösung (700 ml) und dann mit Kochsalzlösung (400 ml) gewaschen. Das Lösungsmittel wird nach dem Trocknen mit Natriumsulfat unter vermindertem Druck entfernt, um ein helles gelbbraunes Öl zu erhalten. Das Rohprodukt wird aus 250 ml Propan-2-ol umkristallisiert. Die Ausbeute beträgt 39,5 g (75%) und die HPLC-Analyse ergab 99,92% Reinheit.
¹H NMR (300MHz, CDCl₃) δ (ppm): 0.91 (3H, t), 1.72 (2H, m), 1.8 (2H, q), 2.2 (4H, m), 2.58 (2H, t), 2.66 (2H, m), 2.94 (2H, t), 3.40 (3H, s), 4.05 (2H, s), 6.72 (1H, d), 6.87 (1H, dd), 7.08 (1H, d), 7.30 (5H, m)
¹³C NMR, δ: 9.48 (CH₃), 27.73 (CH₂), 30.68 (CH₂), 33.06 (2xCH₂), 50.13 (2xCH₂), 59.15 (CH₃O), 59.97 (CH₂N), 61.48 (C), 70.52 (CH₂O), 123.40 (CHₐᵣ), 124.55 (CHₐᵣ), 126.6 (CHₐᵣ), 127.7 (CHₐᵣ), 128.57 (2xCHₐᵣ), 131.32 (2xCHₐᵣ), 141.23 (Cₐᵣ), 142.63 (Cₐᵣ), 174.59 (CO)
MS: m/e =387 (M+1)⁺

### Beispiel 9

### Herstellung von 1-Ethyl-4-(2-(4-methoxymethyl)-4-(phenylamino)piperidin-1-yl)ethyl)-1H-tetrazol-5(4H)-on

Eine Mischung aus 20 g (0,091 mol) 4-Phenylamino-4-methoxymethylpiperidin, 14,4 g (0,082 mol) 1-(2-Chlorethyl)-4-ethyl-1H-tetrazol-5(4H)-on, 19,3 g (0,182 mol) Natriumcarbonat und Kaliumiodid 1,5 g (0,009 mol) in 1300 ml Ethylmethylketon wird 20 h unter Rückfluss gerührt. Die Reaktionsmischung wird auf 25 °C abgekühlt und filtriert. Die organische Lösung wird 3-mal mit 40 ml Wasser extrahiert und über Natriumsulfat getrocknet. Der Feststoff wird abfiltriert und die Lösung zur Trockne eingedampft. Das Rohprodukt wird in 300 ml Ethanol gesättigt mit gasförmiger Salzsäure (15%) gelöst. Das Produkt wird auf 10 °C abgekühlt und für 4 Stunden bei dieser Temperatur gehalten. Die Kristalle werden abfiltriert und unter Vakuum getrocknet. Man erhält 20,3 g (56,4%) 1-Ethyl-4-(2-(4-methoxymethyl)-4-(phenylamino)piperidin-1-yl)ethyl-1H-tetrazol-5(4H)-on Dihydrochlorid mit einer Reinheit von 97,8% (HPLC).
¹H NMR (300MHz, CDCl₃) δ (ppm): 1.43 (3H, t), 1.65 (2H, m), 1.88 (2H, m), 2.65 (4H, m), 2.78 (2H, t), 3.29 (3H, s), 3.29 (2H, s), 3.98 (2H, q), 4.06 (2H, t), 6.81 (3H, m), 7.16 (2H, m)
¹³C NMR, δ: 13.78 (CH₃), 32.66 (2xCH₂), 40.04 (CH₂N), 42.25 (CH₂N), 48.84 (2xCH₂), 54.88 (CH₂N), 55.77 (C), 59.05 (CH₃O), 76.72 (CH₂O), 119.69 (2xCHₐᵣ), 119.72 (CHₐᵣ), 128.81 (2xCHₐᵣ), 145.81 (Cₐᵣ), 150.67 (CO)
MS: m/e =361 (M+1)⁺

### Beispiel 10

### Herstellung von N-{1-[2-(4-Ethyl-5-oxo-4,5-dihydro-1H-tetrazol-1-yl)ethyl]-4-(methoxymethyl)piperidin-4-yl}-N-phenylpropionamid

1-Ethyl-4-(2-(4-methoxymethyl)-4-(phenylamino)piperidin-1-yl)ethyl)-1H-tetrazol-5(4H)-on 20 g (0,055 mol), 150 ml Dichlormethan (CH₂Cl₂) und 15,4 ml (0,111 mol) Triethylamin werden in einem 1 L 3-Hals-Glaskolben vorgelegt. Danach werden 6,3 ml (0,0721 mol) Propionylchlorid tropfenweise zu der Reaktionsmischung über einen Zeitraum von 20 Minuten zugegeben. Nach der Zugabe wird die Reaktionsmischung bei Raumtemperatur für weitere 5 Stunden gerührt. Anschließend wird das Reaktionsgemisch mit 100 ml destilliertem Wasser verdünnt und die organische Phase von der wässrigen Phase getrennt. Die organische Phase wird mit 10% iger Natriumcarbonatlösung (100 ml) und dann mit Kochsalzlösung (100 ml) gewaschen. Nach Trocknung über Natriumsulfat wird das Lösungsmittel im Vakuum entfernt. Das Rohprodukt wird in 100 ml Methanol, das mit gasförmiger Salzsäure (15%) gesättigt ist, gelöst. Das Produkt wird unter Rühren mit Eis abgekühlt bis das Produkt auskristallisiert. Das Produkt wird filtriert und unter Vakuum für 7 Stunden getrocknet. 20,7 g (76,5%) N-{-[2-(4-Ethyl-5-oxo-4,5-dihydro-1H-tetrazol-1-yl)ethyl]-4-(methoxymethyl)piperidin-4-yl}-N-phenylpropionamid Dihydrochlorid. Die HPLC-Analyse ergab 98,6% Reinheit
¹H NMR (300MHz, CDCl₃) δ (ppm): 0.93 (3H, t), 1.43 (3H, t), 1.68 (2H, m), 1.83 (3H, s), 2.25 (4H, m), 2.65 (2H, m), 3.42 (3H, s), 3.98 (2H, q), 3.99 (2H, d), 4.04 (2H, s), 7.32 (5H, m)
¹³C NMR, δ: 9.47 (CH₃), 13.81 (CH₃), 30.68 (2xCH₂), 32.84 (CH₂), 40.09 (CH₂N), 42.20 (CH₂N), 50.00 (2xCH₂), 55.54 (CH₂N), 59.16 (CH₃O), 61.21 (C), 70.56 (CH₂O), 127.85 (CHₐᵣ), 128.63 (2xCHₐᵣ), 131.27 (2xCHₐᵣ), 141.13 (Cₐᵣ), 150.56 (CO), 174.64 (CO)
MS: m/e =417 (M+1)⁺

## Patentansprüche

1. Verwendung von 4-(Phenylamino)-4-(methoxymethyl)piperidin für die Synthese einer Verbindung der Formel I 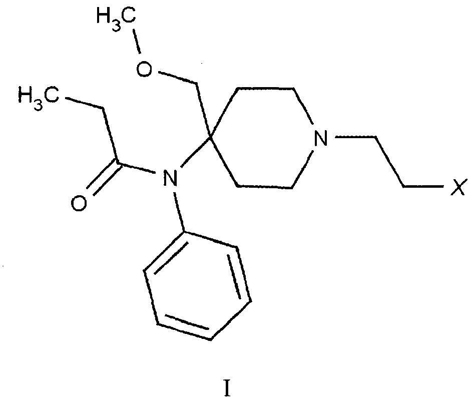 wobei die Verbindung I Sufentanil (mit X = 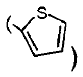 oder Alfentanil (mit X = 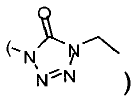 ist.

2. Verfahren zur Herstellung von 4-Methoxymethyl-4(phenylamino)-1-(2-thienyl)-ethylpiperidin, durch Umsetzen von 4-(Phenylamino)-4-(methoxymethyl)piperidin mit einer Verbindung der Formel II, so dass 4-Methoxymethyl-4(phenylamino)-1-(2-thienyl)-ethyl-piperidin gebildet-wird: 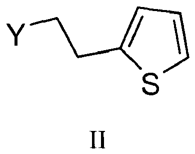 Wobei Y eine Abgangsgruppe darstellt ausgewählt aus Bromid, Iodid, Methansulfonat, p-Toluolsulfonat, Chlorid, und wobei die Umsetzung gegebenenfalls in Gegenwart eines Lösungsmittels durchgeführt wird, dass ausgewählt ist aus Acetonitril, 2-Propanol, 2-Butanol, Butan-2-on, und Isobutylketon und deren Mischungen.

3. Verfahren nach Anspruch 2, wobei Y Methansulfonat ist.

4. Verfahren zur Herstellung von Alfentanil umfassend folgende Schritte:
Umsetzen von 1-Ethyl-4-(2-(4-methoxymethyl)-4-(phenylamino)piperidin-1-yl)ethyl)-1H-tetrazol-5 (4H)-on mit Verbindung III: 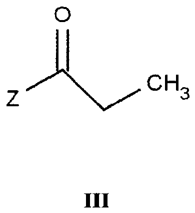
So dass Alfentanil entsteht, wobei Z ausgewählt ist aus -OC(O)-C2H5, Cl, Br, und wobei die Umsetzung gegebenenfalls in Gegenwart eines Lösungsmittels durchgeführt wird, dass ausgewählt ist aus Dimethylformamid, Dichlormethan, Dimethylsulfoxid und Tetrahydrofuran und deren Mischungen.

5. Verfahren nach Anspruch 4, wobei das 1-Ethyl-4-(2-(4-methoxymethyl)-4-(phenylamino) piperidin-1-yl)ethyl)-1H-tetrazol-5(4H)-on gebildet wird durch Umsetzen von 4-(Phenylamino)-4-(methoxymethyl)-piperidin mit einem Alkylierungsmittel ausgewählt aus 1-(2-Chlorethyl)-4-ethyl-1,4-dihydro-5H-tetrazol-5-on, 1-(2-Bromethyl)-4-ethyl-1,4-dihydro-5H-tetrazol-5-on, 1-(2-iodoethyl)-4-ethyl-1,4-dihydro-5H-tetrazol-5-on, und 1-(2-Methansulfonat-ethanoyl)-4-ethyl-1,4-dihydro-5H-tetrazol-5-on, so dass 1-Ethyl-4-(2-(4-methoxymethyl)-4-(phenylamino)piperidin-1-yl)ethyl)-1H-tetrazol-5(4H)-on entsteht , wobei die Umsetzung gegebenenfalls in Gegenwart eines Lösungsmittels durchgeführt wird, dass ausgewählt ist aus Acetonitril, 2-Propanol, 2-Butanol, Butan-2-on, und Isobutylketon und deren Mischungen.

6. Ein Verfahren nach einem der Ansprüche 1 bis 3, wobei die Reaktion unter Atmosphärendruck bei der Siedetemperatur des Reaktionsgemisches durchgeführt wird.

7. Synthese von 4-(Phenylamino)-4-(methoxymethyl)piperidin, umfassend die folgenden Schritte:

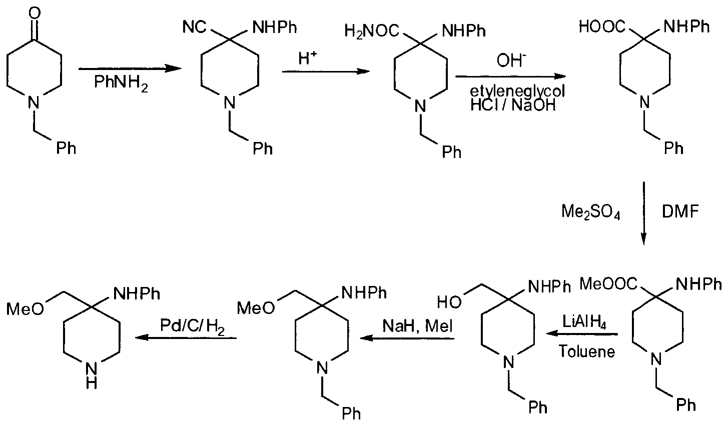

8. Synthese von 4-(Phenylamino)-4-(methoxymethyl)piperidin umfassend die folgenden Schritte:

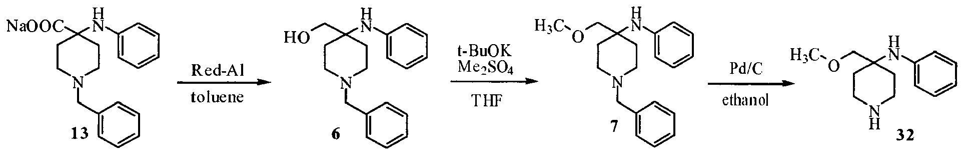

9. Synthese von Sufentanil nach dem folgenden Schema:

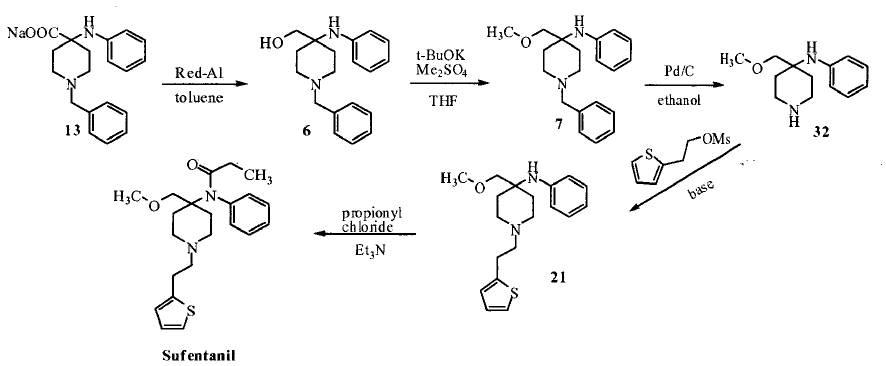

10. Synthese von Alfentanil nach dem folgenden Schema

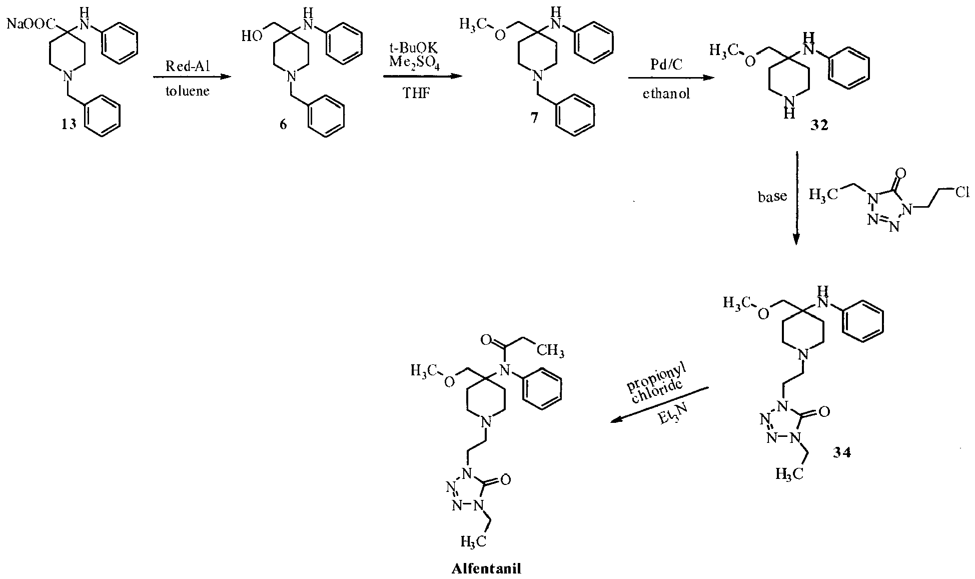

## Claims

1. Use of 4-(phenylamino)-4-(methoxymethyl)piperidine for the synthesis of a compound of formula I 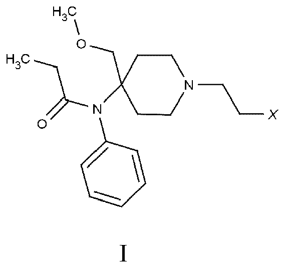 wherein the compound of formula I represents Sufentanil (with X = 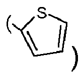 or Alfentanil (with X =

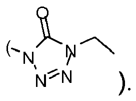

2. A method for producing 4-methoxymethyl-4(phenylamino)-1-(2-thienyl)-ethylpiperidine, obtained by reacting 4 - (phenylamino) -4 - (methoxymethyl) piperidine with a compound of formula II to give 4 -methoxymethyl-4 (phenylamino) -1 - (2-thienyl)-ethylpiperidine. 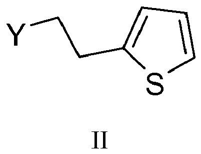 Wherein Y is a leaving group and is selected from bromide, iodide, methanesulfonate, p-toluenesulfonate, chloride, and wherein the reaction is optionally performed in the presence of a solvent that is selected from acetonitrile, 2-propanol, 2-butanol, butan-2-one and isobutylketone and mixtures thereof.

3. A method according to claim 2, wherein Y is methanesulfonate.

4. A process for preparing alfentanil, comprising the following steps:
Reacting 1-methyl-4-(2 - (4-methoxymethyl) -4 - (phenylamino) piperidine-1-yl) ethyl)-1H-tetrazol-5(4H)-one with a compound III: 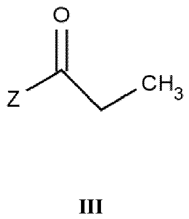
In a way that alfentanil is produced, wherein Z is selected from-OC (O)-C₂H₅, Cl, Br, and wherein the reaction is optionally performed in the presence of a solvent that is selected from dimethylformamide, dichloromethane, dimethylsulfoxide, tetrahydrofuran and their mixtures.

5. A method according to claim 4, wherein the 1-methyl-4-(2 - (4-methoxymethyl) -4 - (phenylamino) piperidine-1-yl) ethyl)-1H-tetrazol-5 (4H)-one is formed by reacting 4 - (phenylamino) -4 - (methoxymethyl) piperidine with an alkylating agent selected from 1 - (2-chloroethyl)-4-ethyl-1,4-dihydro-5H-tetrazol-5-one, 1-(2-bromoethyl)-4-ethyl-1,4-dihydro-5H-tetrazol-5-one, 1-(2-iodoethyl)-4-ethyl-1,4-dihydro-5H-tetrazol-5-one and 1-(2-methanesulfonate-ethanoyl)-4-ethyl-1,4-dihydro-5H-tetrazol-5-one to obtain 1-ethyl-4-(2 - (4-methoxymethyl) - 4 - (phenylamino) piperidine-1-yl)ethyl)-1H-tetrazol-5 (4H)-one, wherein the reaction is optionally carried out in the presence of a solvent that is selected from acetonitrile, 2-propanol, 2-butanol, butan-2-one and isobutylketone and mixtures thereof.

6. A method according to any one of claims 1 to 3, wherein the reaction is carried out under atmospheric pressure at the boiling point of the reaction mixture.

7. Synthesis of 4 - (phenylamino) -4 - (methoxymethyl) piperidine comprising the steps:

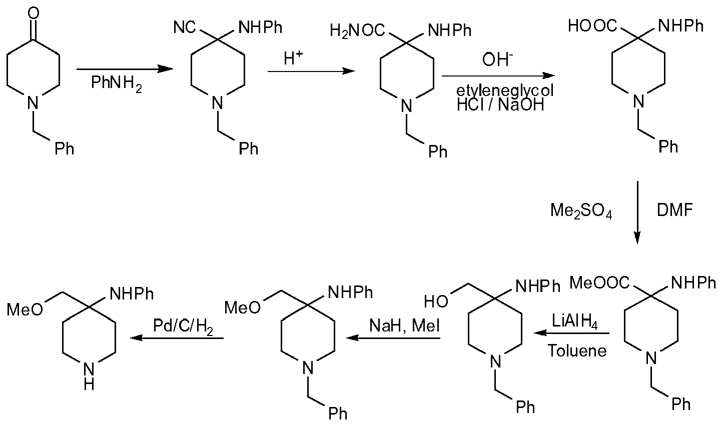

8. Synthesis of 4 - (phenylamino) -4 - (methoxymethyl) piperidine comprising the steps:

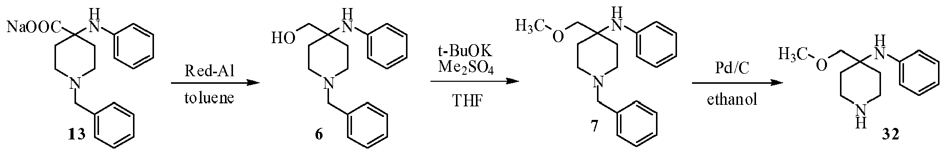

9. Synthesis of Sufentanil according to the following scheme:

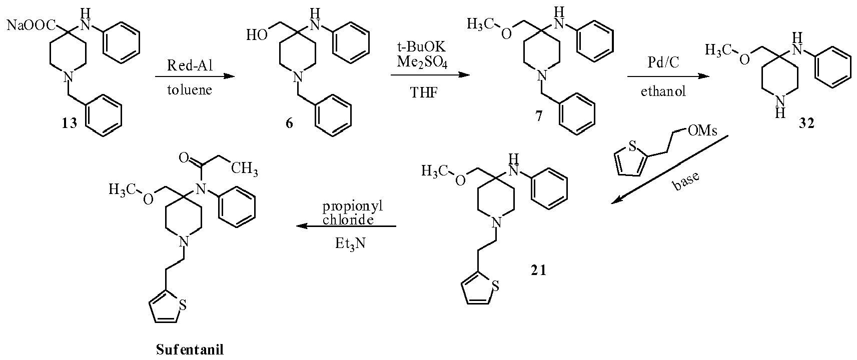

10. Synthesis of Alfentanil according to the following scheme:

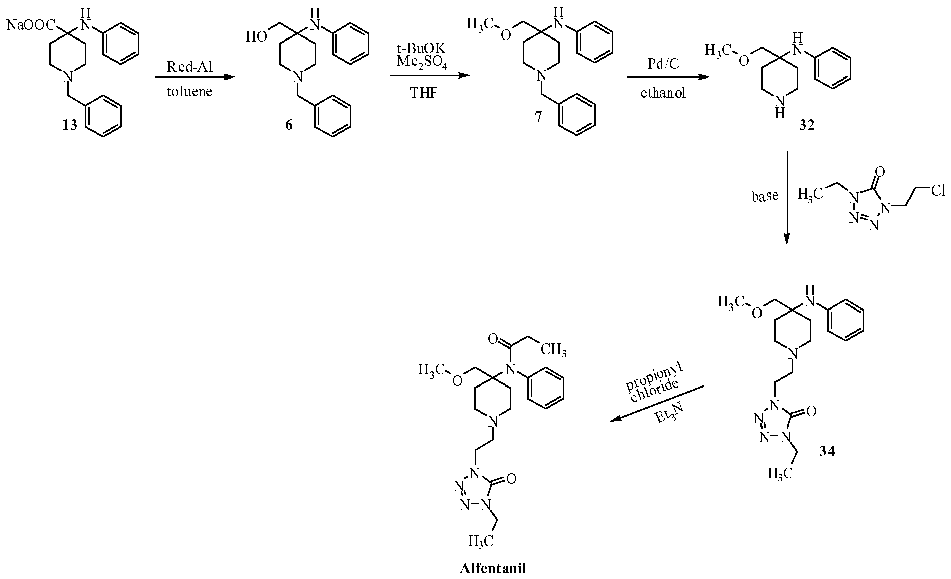

## Revendications

1. L'utilisation de 4-(phénylamino)-4-(méthoxyméthyl) pipéridine dans la synthèse d'un composé de formule I, 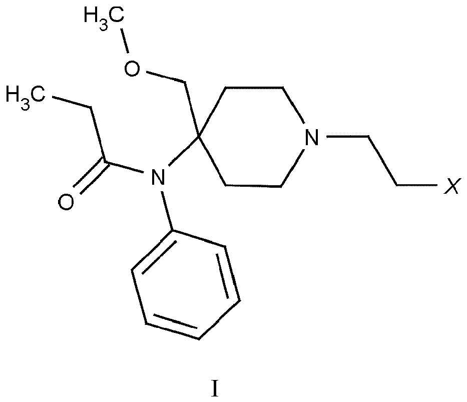 dans laquelle le composé I soit le sufentanil (avec X = 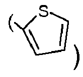 ou l'alfentanil (avec X =

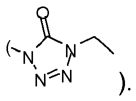

2. Procédé de préparation de 4-méthoxyméthyl-4 (phénylamino)-1-(2-thiényl)-éthyl-piperidine, obtenue en faisant réagir la 4-(phénylamino)-4-(méthoxyméthyl) pipéridine avec un composé de formule II, pour conduire à la 4-méthoxyméthyl-4(phénylamino)-1-(2-thiényl)-éthyl-piperidine. 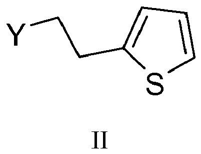 Dans lequel Y est choisi comme groupe partant dans le groupe consistant en bromure, iodure, méthanesulfonate, p-toluènesulfonate, chlorure et dans lequel la réaction est éventuellement effectuée en présence d'un solvant qui est choisi parmi l'acétonitrile, le 2-propanol, le 2-butanol, le butan-2-one, l'isobutylcétone et des mélanges de ceux-ci.

3. Procédé selon la revendication 2, dans lequel Y est méthanesulfonate.

4. Procédé de préparation d'alfentanil comprenant les étapes suivantes:
Faire réagir le 1-éthyl-4-(2-(4-méthoxyméthyl)-4-(phénylamino) piperidine-1-yl) éthyl)-1H-tétrazol-5 (4H)-one avec un composé de formule III: 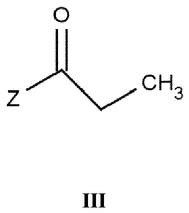
pour conduire à l'alfentanil. Dans ce procédé Z représente un groupement -OC(O)-C₂H₅, Cl ou Br et la transformation est éventuellement effectué en présence d'un solvant, qui est choisi parmi le diméthylformamide, le dichlorométhane, le diméthoxysulfoxyde, le tétrahydrofurane et leurs mélanges.

5. Procédé selon la revendication 4 dans lequel le 1-éthyl-4(2-(4-méthoxyméthyl)-4-(phénylamino)pipéridine-1-yl)éthyl)-1H-tétrazol-5(4H)one est formé en faisant réagir le 4-(phénylamino)-4-(méthoxyméthyl)pipéridine avec un agent alkylante qui est choisi parmi le 1-(2-chloro-éthyl)-4-éthyl-1,4-dihydro-5H-tétrazol-5-one, le 1-(2-bromoéthyl)-4-éthyl-1,4-dihydro-5H-tétrazol-5-one, le 1-(2-iodoéthyl)-4-éthyl-1,4-dihydro-5H-tétrazol-5-one et le 1-(2-méthanesulfonate-ethanoyl)-4-éthyl-1,4-dihydro-5H-tétrazol-5-one de façon que le 1-éthyl-4(2-(4-méthoxyméthyl)-4-(phénylamino)pipéridine-1-yl) éthyl)-1H-tétrazol-5(4H)-one est formé et dans lequel la réaction est éventuellement effectué en présence d'un solvant qui est choisi parmi l'acétonitrile, le 2-propanol, le 2-butanol, le 2 butan-2-one et l'isobutylcétone et des mélanges de ceux-ci.

6. Un procédé selon l'une quelconque des revendications 1 à 3, dans lequel la réaction est effectuée sous la pression atmosphérique au niveau de point d'ébullition du mélange réactionnel.

7. Synthèse du 4-(phénylamino)-4-(méthoxyméthyl)pipéridine comprenant les étapes consistant à:

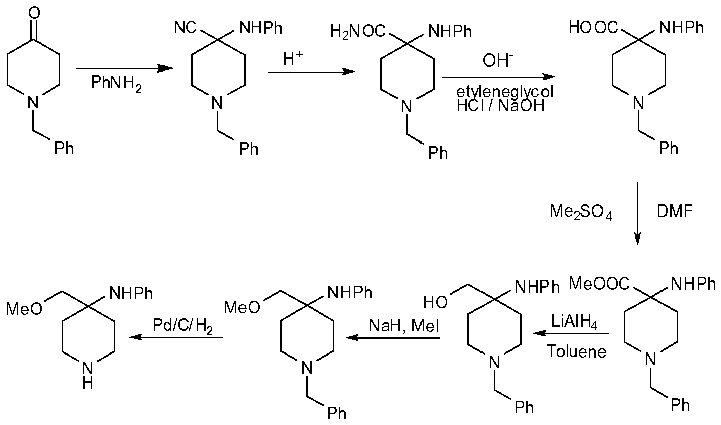

8. Synthèse du 4-(phénylamino)-4-(méthoxyméthyl) pipéridine comprenant les étapes consistant à:

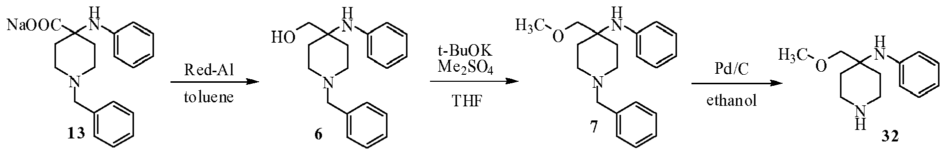

9. Synthèse de sufentanil par le schème suivant:

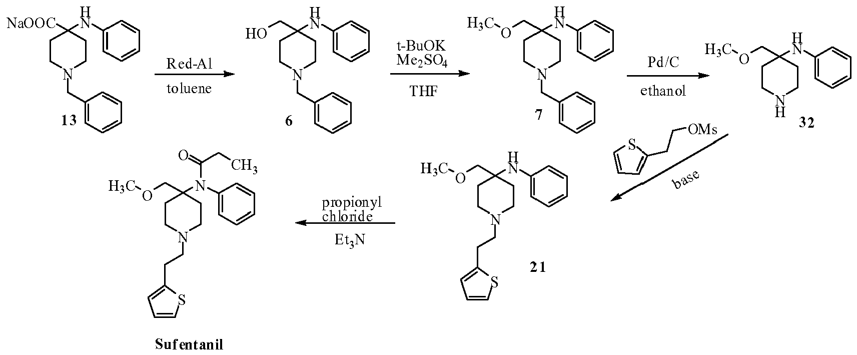

10. Synthèse d'alfentanil par le schème suivant:
